# EUROPEAN PATENT SPECIFICATION

(11) **EP 1 494 580 B1**
(45) Date of publication and mention of the grant of the patent: **23.01.2008**
(21) Application number: 03702980.8
(22) Date of filing: 06.03.2003
(51) Int. Cl.: A61B 5/0408, H01B 1/12

(54) **A WEARABLE MONITORING SYSTEM AND METHOD OF MANUFACTURING OF A WEARABLE MONITORING SYSTEM**
EIN TRAGBARES ÜBERWACHUNGSSYSTEM UND HERSTELLUNGSMETHODE FÜR EIN TRAGBARES ÜBERWACHUNGSSYSTEM
SYSTEME DE SURVEILLANCE POUVANT ETRE PORTE ET PROCEDE DE FABRICATION ASSOCIE

(30) Priority: 29.03.2002 EP 02076226
(43) Date of publication of application: 12.01.2005
(73) Proprietor: Koninklijke Philips Electronics N.V., 5621 BA Eindhoven (NL); Philips Intellectual Property & Standards GmbH, 20099 Hamburg (DE)
(72) Inventor: LAUTER, Josef, NL-5656 AA Eindhoven (NL); REITER, Harald, NL-5656 AA Eindhoven (NL); SCHMIDT, Ralf, NL-5656 AA Eindhoven (NL); SUCH, Olaf, NL-5656 AA Eindhoven (NL); VOGTMEIER, Gereon, NL-5656 AA Eindhoven (NL); REICHINGER, Christian, NL-5656 AA Eindhoven (NL)
(74) Representative: Schouten, Marcus Maria
(86) International application number: PCT/IB2003/000850
(87) International publication number: WO 2003/082103

(56) References cited:
- FR-A- 2 531 330
- US-A- 3 808 678
- US-A- 3 998 213
- US-A- 4 202 344

## Description

The invention relates to a method for manufacturing a wearable monitoring system, said method comprising a step of providing a fabric-based elastic belt for housing of electrodes.

The invention further relates to a system for cardiac monitoring.

A method for manufacturing a wearable monitoring system is known from WO 98/41279. According to the known method a textile garment is provided with monitoring electrodes, said electrodes being sewn, embroidered, embedded or attached to the garment by means of an adhesive.

A monitoring system of the type known from the prior art can be used in the field of monitoring a physiological activity of a patient, for example a heart rate. In this case it is of an importance that the monitoring system is user-friendly and can be worn by the patient during long periods of time without the patient experiencing any discomfort. It is further important that the number of electrodes is minimized in such a monitoring system and that the electrode system is durable and reliable.

FR-A-2 531 330 discloses a fabric-based elastic belt with monitoring electrodes for heart signals. A pin on the metal electrodes is stuck through a rubber plate mounted on the belt but nothing traverses the belt itself.

It is an object of the invention to provide a method for manufacturing of a wearable monitoring system, where the electrodes are reliably attached to the garment and where the manufacturing costs are reduced.

The method according to the invention is characterized in that the electrodes are provided by molding an electrode material through the elastic belt. It has been found that by applying a per se known molding operation to attach an electrode to the belt of the garment, for example an underwear slip or a brassier a reliable and cheap monitoring system can be produced. The fact that the necessary electrodes are integrated in the belt of the garment contributes to the user-friendliness of such a monitoring system. Furthermore such a system is washable which further contributes to the comfort of the patient. Due to the fact that the electrodes are integrated with the elastic band, which is flexible during body movements, the position of the electrodes with respect to the patient's skin, is not fixed. Thus skin irritation is minimized. A minimizing of the motion artifacts of the measurements is achieved by an adapted pressure on the electrodes, which is generated from the tension in the elastic band. A preferable tension for the elastic band is chosen in the interval from 12 to 24 N. It is found that the applied tension further facilitates sweating beneath the electrodes, improving the electrical conductivity and thus, leading to a noise reduction. Due to the fact that in general people are accustomed to experience tension in the waist region, it will not lead to a patient discomfort. Additionally, the small size of the electrodes further contributes to the user-friendliness of the monitoring system.

An embodiment of the method according to the invention is characterized in that for the step of providing the electrodes use is made of a mould including electrical wiring for providing electrical connections to the electrodes, said wiring being permanently attached to the electrodes after the mould is released. According to this technical measure the necessary wiring can also be integrated in the belt, further contributing to the patient's comfort and the durability of the monitoring system. The wire material can comprise conventional copper wires or, alternatively, it can comprise electrically conductive silicon bands. The silicon bands can be appropriately chosen so that to match the elasticity of the elastic band.

An embodiment of the method according to the invention is characterized in that the electrode material comprises conductive rubber and in that during the molding a vulcanization of the conductive rubber is performed. It is advantageously to use a conductive rubber for manufacturing the electrodes, as the rubber is stretchable thus contributing to the user-friendliness of the system. It has been found that by applying a per se known vulcanizing operation (pressing and heating) to attach the electrode material to the belt of the garment a reliable and cheap monitoring system can be produced.

A further embodiment of the method according to the invention is characterized in that for a wiring a material is used which has a substantially the same elasticity as the material of the elastic belt. This technical measure ensures approximately equal stretchability of the elastic belt, further contributing to the reliability of the monitoring system. Conductive rubbers are available which are well-suited for these purposes.

The invention further relates to a monitoring system, characterized in that the monitoring system is a cardiac monitoring system. It has been established that particularly patients with abnormalities in the heart rate require a continuous monitoring. The system manufactured according to the method presented above is very convenient as it can be worn as regular underwear garment without causing extra discomfort to the patient. Such a monitoring system can be further adapted to produce a local and/or remote alarm in case a life threatening abnormality occurs. For this purpose this monitoring system can be integrated in a state of the art alarm system, known, for example in the field of cardiac arrest monitoring.

An embodiment of the monitoring system is characterized in that said system comprises at least two electrodes. It has been found out that for purposes of cardiac arrest monitoring it is sufficient to provide two electrodes. These electrodes can be integrated in the abdominal belt of the underwear or in the thoracic belt of the brassier. For the ECG monitoring at least three electrodes suffice.

A still further embodiment of the monitoring system according to the invention is characterized in that said system further comprises a motion sensor. This technical feature has the advantage that in case the monitoring system detects a cardiac arrest this condition is double-checked by means of a motion detector. This additional feature reduces a chance of false alarms.

These and other aspects of the invention will be discussed with reference to the figures.
Fig. 1 presents a schematic view of an embodiment of the monitoring system.
Fig. 2 presents schematically an embodiment of a cross-section of one sensor attached to the elastic belt according to the method of the invention.

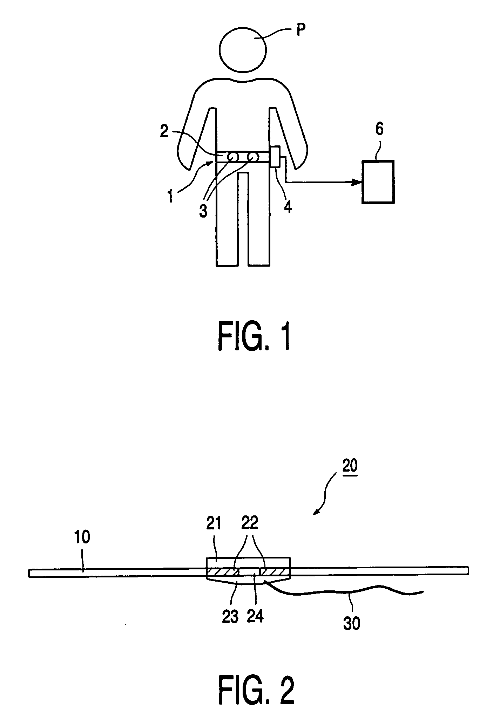

Fig. 1 presents a schematic view of the wearable monitoring system 1 according to the invention. A patient P is supplied with a set of sensors 3 integrated on the elastic belt 2, the sensors being attached to the elastic belt according to the method of the invention. The set of sensors 3 are thus reliably positioned in a contact with the patient's skin in order to acquire a desirable physiological signal. For example, such a monitoring system can be arranged to perform measurements of the heart activity or a signal derived from the heart activity. The sensors 3 comprising electrodes (not shown) are electrically connected to the storage and analysis device (SAD) 4. The SAD can be arranged to perform a primary data analysis in order to interpret the acquired physiological data, or it can serve as an intermediate data collection station. In the former case the control signal, for example an alarm can be sent to a remote station 6 in case the primary data analysis detects an abnormality. In the latter case the data is being sent for analysis at the remote station 6. The latter case can be advantageous in situations where voluminous data is to be analyzed for different purposes. It is found that in order to detect the event of the cardiac arrest it is sufficient to use only two sensors integrated in the belt. Next to these sensors a motion detector can be assembled on the elastic belt. Any known portable motion detector can be used for this purpose. In a case of the cardiac arrest event the patient is not moving, which is conferred by the motion sensor. In this case (no patient movement) it is found to be feasible to measure the ECG with two sensors localized in the abdominal area of the patient.

Fig. 2 presents schematically a cross-section of one sensor, comprising an electrode assembly 20 attached to the elastic belt 10 according to the method of the invention. The electrode material 20 can be manufactured from a mixture of electrically conductive graphite with a silicon gel, for example. Other suitable materials can also be used to manufacture the electrode, for example conductive rubbers. In case a conductive rubber is used the electrode body can be attached to the elastic belt by means of the per se known vulcanization procedure comprising the steps of heating and pressing. It is also possible that the elastic belt is pre-processed to comprise cut-aways in areas where the electrodes are to be positioned. As is shown in Fig. 2, a part of the electrode 24 is located in such a cut-away of the belt and another part 22 of the electrode is joined with the elastic belt 10. The outer body of the electrode can be covered by a mould [not shown] at the backside surface 23 of the electrode. The electrical connections to the electrode 20 of the sensor are realized by means of a wire, schematically presented by 30. The wire 30 leads to other electrodes in the monitoring system and to the motion sensor [optional] and to the Storage and Analysis Device [not shown] also attached to the belt. Thus, a cheap, durable and reliable wearable monitoring system can easily be realized using the method of the invention.

## Claims

1. A method for manufacturing a wearable monitoring system, said method comprising a step of providing a fabric-based elastic belt (2) for housing of electrodes (3), **characterized in that** the electrodes are provided by molding of an electrode material (22) through the elastic belt.

2. A method according to claim 1, **characterized in that** for the step of providing the electrodes use is made of a mould including electrical wiring for providing electrical connections to the electrodes, said wiring being permanently attached to the electrodes after the mould is released.

3. A method according to claims 1 or 2 **characterized in that** the electrode material comprises conductive rubber and **in that** during the molding a vulcanization of the conductive rubber is performed.

4. A method according to claim 2 or 3, **characterized in that** for the wiring a material is used which has a substantially the same elasticity as the material of the elastic belt.

5. A method according to claim 4, **characterized in that** the material for wiring is a conductive rubber.

6. A wearable cardiac monitoring system comprising a fabric-based elastic belt housing electrodes and **characterised in that** the system is obtained by the method according to any one of the preceding claims.

7. A monitoring system according to claim 6, **characterized in that** said system comprises at least two electrodes.

8. A monitoring system according to one of the preceding claims 6 or 7, **characterized in that** said system further comprises a motion sensor.

## Patentansprüche

1. Verfahren zur Herstellung eines tragbaren Überwachungssystems, wobei das genannte Verfahren einen Schritt des Schaffens eines stoffbasierten elastischen Gurts (2) zur Aufnahme von Elektroden (3) umfasst, **dadurch gekennzeichnet, dass** die Elektroden durch Pressen eines Elektrodenmaterials (22) durch den elastischen Gurt geschaffen werden.

2. Verfahren nach Anspruch 1, **dadurch gekennzeichnet, dass** für den Schritt des Schaffens der Elektroden eine Pressform verwendet wird, die elektrische Verdrahtung zum Schaffen elektrischer Verbindungen zu den Elektroden enthält, wobei die genannte Verdrahtung permanent an die Elektroden angeschlossen ist, nachdem die Pressform abgelöst ist.

3. Verfahren nach Anspruch 1 oder 2, **dadurch gekennzeichnet, dass** das Elektrodenmaterial leitendes Gummi umfasst und dass während des Pressens eine Vulkanisierung des leitenden Gummis durchgeführt wird.

4. Verfahren nach Anspruch 2 oder 3, **dadurch gekennzeichnet, dass** für die Verdrahtung ein Material verwendet wird, das im Wesentlichen dieselbe Elastizität hat wie das Material des elastischen Gurts.

5. Verfahren nach Anspruch 4, **dadurch gekennzeichnet, dass** das Material für die Verdrahtung ein elastisches Gummi ist.

6. Tragbares Herzüberwachungssystem mit einem stoffbasierten elastischen Gurt, der Elektroden aufnimmt, **dadurch gekennzeichnet, dass** das System mit dem Verfahren nach einem der vorhergehenden Ansprüche erhalten wird.

7. Überwachungssystem nach Anspruch 6, **dadurch gekennzeichnet, dass** das genannte System mindestens zwei Elektroden umfasst.

8. Überwachungssystem nach einem der vorhergehenden Anspruch 6 oder 7, **dadurch gekennzeichnet, dass** das genannte System weiter einen Bewegungssensor umfasst.

## Revendications

1. Procédé de fabrication d'un système portable de contrôle, ledit procédé comprenant une étape consistant à fournir une ceinture élastique à base de tissu (2) pour incorporer des électrodes (3), **caractérisé en ce que** les électrodes sont appliquées par moulage d'un matériau d'électrode (22) à travers la ceinture élastique.

2. Procédé selon la revendication 1, **caractérisé en ce que** pour l'étape consistant à fournir les électrodes on fait usage d'un moule y compris du câblage électrique pour réaliser des connexions électriques aux électrodes, ledit câblage étant fixé en permanence aux électrodes après le dégagement du moule.

3. Procédé selon la revendication 1 ou 2, **caractérisé en ce que** le matériau d'électrode comprend du caoutchouc conducteur et **en ce que** pendant le moulage on exécute une vulcanisation du caoutchouc conducteur.

4. Procédé selon la revendication 2 ou 3, **caractérisé en ce que** pour le câblage on fait usage d'un matériau qui présente sensiblement la même élasticité que le matériau de la ceinture élastique.

5. Procédé selon la revendication 4, **caractérisé en ce que** le matériau pour le câblage est un caoutchouc conducteur.

6. Système portable de contrôle cardiaque comprenant une ceinture élastique à base de tissu incorporant des électrodes et étant **caractérisé en ce que** le système est obtenu par le procédé selon l'une quelconque des revendications précédentes 1 à 5.

7. Système de contrôle selon la revendication 6, **caractérisé en ce que** ledit système comprend au moins deux électrodes.

8. Système de contrôle selon une des revendications précédentes 6 ou 7, **caractérisé en ce que** ledit système comprend encore un détecteur de mouvement.
